# EUROPÄISCHE PATENTANMELDUNG

(11) **EP 0 592 936 A1**
(43) Veröffentlichungstag der Anmeldung: **20.04.1994**
(21) Anmeldenummer: 93116165.7
(22) Anmeldetag: 06.10.1993
(51) Int. Cl.: C12M 3/04, C12M 3/06

(54) **Verfahren und Vorrichtung zur Züchtung von Zellen**

(30) Priorität: 09.10.1992 DE 4234109
(71) Anmelder: Dr. Rentschler Biotechnologie GmbH, D-88471 Laupheim (DE)
(72) Erfinder: Wolf, Wieland, Dr., D-88471 Laupheim (DE); Russ, Kurt, D-88477 Orsenhausen (DE); Pakos, Vlademar, Dr., D-88471 Laupheim (DE)
(74) Vertreter: Schwabe - Sandmair - Marx

(57) **Zusammenfassung**

Die Erfindung betrifft ein Verfahren zur Kultivierung von Zellen höherer Organismen in kommunizierenden Kammersystemen, bei dem man die zu kultivierenden Zellen in das Kammersystem einbringt und darin züchtet, das dadurch gekennzeichnet ist, daß man die erforderlichen Kulturbedingungen durch gleichmäßige Oberflächenbegasung herstellt, und wobei man die Begasung durch eine in das Kammersystem eingebrachte mikroporöse Gasversorgungsleitung erzielt.

## Beschreibung

Die Erfindung betrifft das in den Ansprüchen 1 und 2 angegebene Verfahren zur Kultivierung von Zellen höherer Organismen in kommuniziuerenden Kammersystemen sowie eine Vorrichtung zur Züchtung von Zellen höherer Organismen im Labor- und technischen Maßstab nach den Ansprüchen 3 bis 6. Die rasche Entwicklung der Biotechnologie erfordert möglichst einfache, aber wirtschaftliche Verfahren zur Kultivierung von Zellen höherer Organismen für die Gewinnung spezieller Zellkulturprodukte. Beispiele für derartige Produkte sind therapeutisch und diagnostisch bedeutsame Substanzen wie Interferon, TPA, Insulin, monoklonale Antikörper etc.. Diese Substanzen finden heute bereits eine breite Anwendung in Therapie und Diagnostik.

Für die Kultivierung dieser die Substanzen produzierenden Zellen gibt es verschiedene Techniken. Neben Suspensions- und Mikroträgerkulturen im Bioreaktor kommt konventionellen Techniken mit sogenannten stationären Kulturen eine besondere Bedeutung zu. Dabei hat sich gezeigt, daß im Vergleich zu einfachen T- oder Rollerflaschen, Kammersysteme, die über mehrere gemeinsam zu befüllende oder entleerende Kompartimente verfügen, eine vorteilhafte Weiterentwicklung darstellen. Mit diesen Systemen kann insbesondere für oberflächenahärent wachsende Zellinien auf technisch einfache Weise eine große Wachstumsfläche bereitgestellt werden. Durch ihre einfache Konstruktion und Handhabbarkeit bilden diese Systeme innerhalb bestimmter Maßstabsgrenzen auch eine Alternative zu den wesentlich komplexeren und teureren Bioreaktoren. Ein derartiges System ist zum Beispiel aus der DE-PS 26 24 047 bekannt. Diese Systeme sind aufgrund der rationelleren Handhabung ökonomischer als die vorhergenannten T- oder Rollerflaschen.

Ein Nachteil derartiger Kammersysteme besteht allerdings darin, daß die Kultivierungsbedingungen nur in sehr engen Grenzen gesteuert werden können und damit die Produktausbeute an den gewünschen Substanzen gering sein kann.

Insbesondere bei schnellwachsenden und stark metabolisierenden Kulturen besteht das Hauptproblem in der Versorgung mit Sauerstoff und in der Übersäuerung des Nährmediums durch Kohlensäure, die aus dem Kohlendioxid des Zellstoffwechsels gebildet wird.

In der Praxis hat sich gezeigt, daß das Wachstum und die Proteinsynthese in derartigen Kammersystemen durch den verfügbaren, über die Flüssigkeitsoberfläche in das Nährmedium diffundierenden Sauerstoff limitiert ist. Einfache Vonrichtungen zur Verbesserung der Sauerstoffversorgung durch Einpumpen eines geeigneten Gasgemisches haben den Nachteil, daß die einzelnen Kultivierungskammern nicht gleichmäßig mit Sauerstoff versorgt werden, was zu ungleichmäßigem Zellwachstum und in der Folge zu niedrigeren Ausbeuten führt. Dabei können auch geringerwertige Produkte gebildet werden, die für eine weitere wirtschaftliche Verwertung nicht geeignet sind.

Die Anmelderin hat nun gefunden, daß sich die vorgenannten Nachteile beseitigen lassen, indem man in derartige Kammersysteme eine das Zellwachstum optimal ermöglichende Gasmischung mittels einer mikroporösen Versorgungsleitung einbringt. Diese Versorgungsleitung kann beispielsweise eine Hohlmembran in Form eines Schlauches oder Stabes sein. Wesentlich für die Versorgungsleitung ist lediglich, daß sie bei Anlegen von Druck über die gesamte Länge im Kammersystem das Gasgemisch gleichmäßig abgibt. Als Versorgungsleitung geeignete und im Handel erhältliche Materialien sind z. B. mikroporöse Membranschläuche aus dem Material 'Accurel^{R}' oder die 'Gore-Tex-Tubes'. Es ist lediglich von Bedeutung, daß durch das mikroporöse Membranmaterial dem freien Gasaustausch zwischen dem Inneren der Versorgungsleitung und dem Kammersystem ein Widerstand entgegengesetzt wird, der durch Erhöhung des Innendruckes der Versorgungsleitung teilweise überwunden werden kann. Das in das Kammersystem übertretende Gasvolumen kann dabei durch Wahl der Porengröße, des Schlauchdurchmessers und des Druckgefälles eingestellt werden.

Gegenstand der Erfindung ist ein Verfahren zur Züchtung von Zellen höherer Organismen in kommunizierenden Kammersystemen, bei dem man die zu kultivierenden Zellen in das Kammersystem einbringt und die Züchtung durchführt, dadurch gekennzeichnet, daß man die erforderlichen Kulturbedingungen durch gleichmäßige Oberflächenbegasung mittels einer in das Kammersystem eingebrachten mikroporösen Gasversorgungsleitung erzielt.

Ein weiterer Gegenstand der vorliegenden Erfindung ist weiterhin eine Vorrichtung zur Kultivierung von Zellen höherer Organismen in kommunizierendden Kammersystemen, dadurch gekennzeichnet, daß die Vorrichtung eine mikroporöse Gasversorgungsleitung aufweist.

Mit dem erfindungsgemäßen Verfahren kann der durch den Zellstoffwechsel bedingte Sauerstoffverbrauch und die übersäurerung des Nährmediums durch das von den Zellen abgegebenes Kohlendioxid kompensiert werden. Dafür wird vorzugsweise ein Gasgemsich verwendet, das zwischen 10 und 50 Vol.% Kohlendioxid enthält. Der verbleibende Volumenanteil wird mit dem physiologisch weitgehend inerten Stickstoffgas aufgefüllt.

Das Konzentrationsgefälle zwischen Flüssigkeits- und Gasphase führt zu Diffusionsvorgängen, bei denen die Moleküle von einem Ort mit höherer Teilchenkonzentration zu einem Ort mit niederer Konzentration bewegt werden. Die Transportarbeit entstammt dabei der Bewegungsenergie der Moleküle. So tritt beispielsweise ein Teil der gelösten Kohlensäure dem Konzentrationsgradienten folgend in Form von Kohlendioxid in die Gasphase über. Umgekehrt wandern Sauerstoffmoleküle aus dem Bereich der höheren Konzentration, der Gasphase, in den Bereich der niedrigeren Konzentration, die Flüssigphase. Durch genaue Dosierung des Kohlendioxid- und des Sauerstoffanteiles in der Gasmischung kann damit der Säuregrad (pH) und die Sauerstoffsättigung (pO2) des Kulturmediums in dem für die Zellen günstigsten Bereich gehalten werden. Dabei gehen als weitere Parameter auch die jeweiligen Diffusionskoeffizienten, und die vom Gas zurückaulegende Wegstrecke ein. Während die Diffusion als Transportmechanismus in erster Linie im Bereich des Phasenüberganges von Bedeutung ist, wird der weitere Transport der Gase im wesentlichen durch thermische Konvektionsvorgänge bewirkt. Im Grenzflächenbereich von Gas und Flüssigkeit können sich Mikrogradienten aufbauen, die das Konzentrationsgefälle und auf diese Weise auch den Gasaustausch reduzieren. Eine Erhöhung des Gasvolumenstromes und damit auch der Konvektion kann bei Bedarf den Aufbau solcher Gradienten verhindern und so zu einer Verbesserung des Gasaustausches an der Grenzfläche führen.

Die jeweils zu übertragenden Gasmengen sind darüberhinaus spezifisch von den Stoffwechselleistungen der jeweils kultivierten Zellinie abhängig und liegen beispielsweise bei diploiden Fibroblasten in der Größenordnung von 0,05 mmol O₂/(10⁹ Zellen · h), bei transformierten Zellen jedoch auch erheblich darüber.

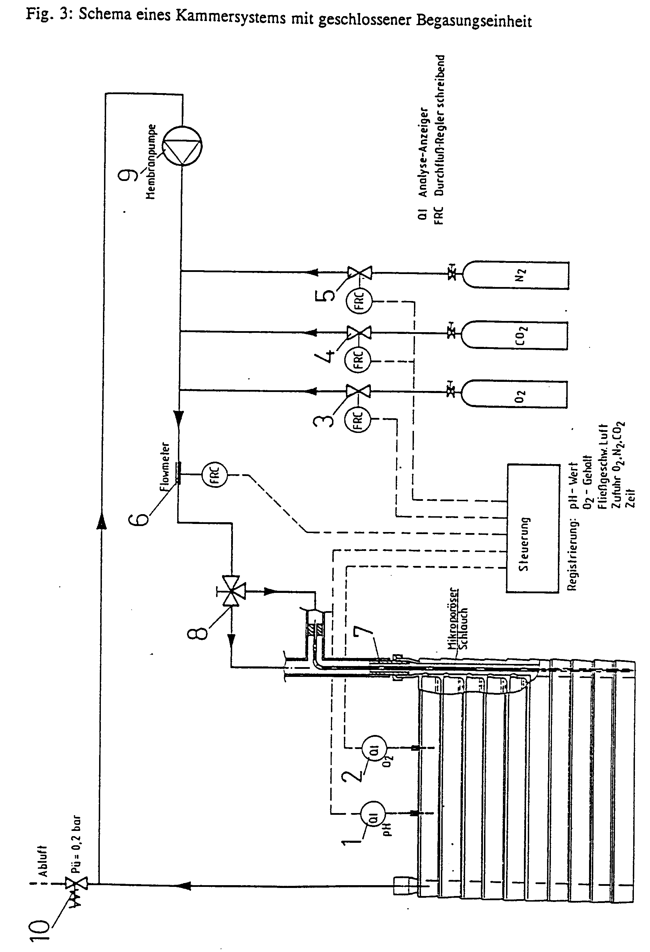

Zur Erreichung eines optimal konfigurierten Systems muß daher die Porenweite und der Durchmesser des mikroporösen Schlauches so gewählt werden, daß einerseits ein genügender Gasvolumendurchsatz gewährleistet ist und sich andererseits der auf der Schlauchinnenseite erforderliche Gegendruck aufbaut. In der Praxis haben sich Porenweiten zwischen 0,2 µm und 4,0 µm und Schlauchdurchmesser zwischen 2 mm und 10 mm bewährt. Je nach Anwendungssystem und Schlauchmaterial können jedoch auch andere Porenweiten und Durchmesser vorteilhaft sein. Die Druckdifferenz zwischen dem Schlauchinneren und dem Kammersystem kann im Prinzip beliebig hoch eingestellt werden, wobei sich in der Praxis allerdings eine niedrige Differenz von 0,1 bis 0,5 bar bewährt hat. Der Zusammenhang zwischen Innendruck und Gasdurchsatz bei einem handelsüblichen Schlauch ist in Fig. 2 dargestellt.

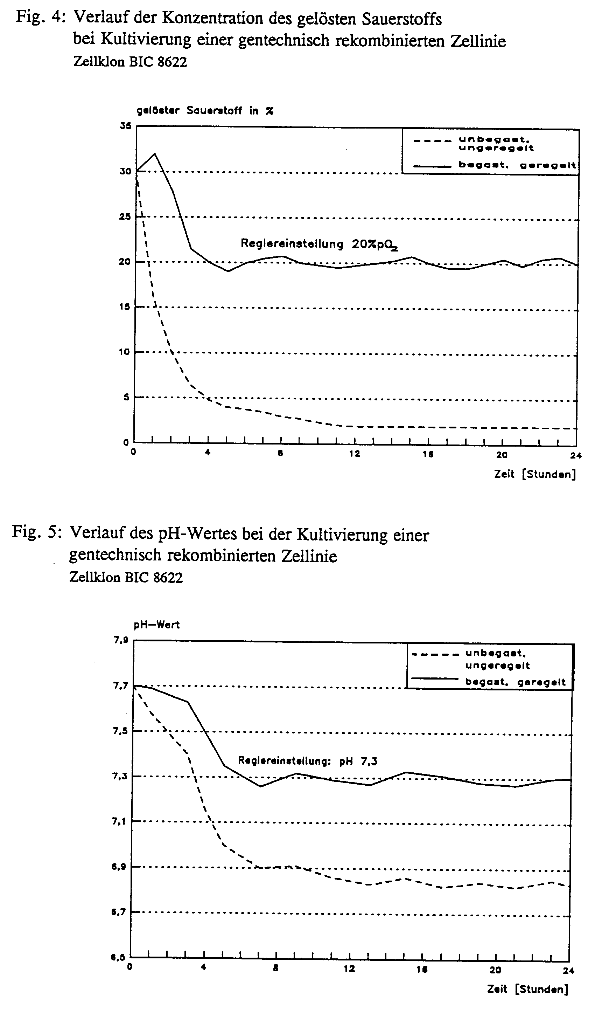

Als besonders vorteilhaft in Bezug auf die Feinregulierung von Sauerstoffsättigung und Säuregrad im Nährmedium, sowie zur Einsparung von Prozeßgasen hat sich die Konstruktion eines geschlossenen Systems erwiesen. Dabei wird das Gasgemisch zirkuliert und über eine entsprechende Meß- und Regeleinheit nur der verbrauchte Sauerstoff ersetzt bzw. das überschüssige Kohlendioxid ausgetragen. Ein derartiges geschlossenes Sytem ist beispielsweise in Figur 3 schematisch dargestellt. Durch Einbringung von Meßsonden z.B. zur Messung des gelösten Sauerstoffes und des Säuregrades (pH-Wertes) im Zellkulturmedium einer oder mehrerer repräsentativer Kammern des Systems können Grenzwerte definiert und gemessen werden, bei deren Über- oder Unterschreitung die Zusammensetzung des Gasgemisches und bei Bedarf auch der Volumenstrom den Erfordernissen entsprechend geregelt werden können. Typische Meßdiagramme eines unbegasten sowie eines begasten und geregelten Kammersystems werden in Beispiel 4, Fig. 4 und 5, gezeigt.

Wird das Begasungssystem erst nachträglich in ein handelsübliches Mehrkammersystem eingebracht, so kann die Montage eines mikroporösen Schlauches durch eine makroporöse oder halbschalenartige Ummantelung, die zur Versteifung des flexiblen Schlauches dient, deutlich erleichtert werden. Bei Verwendung eines mikroporösen Hohlstabes entfällt wegen dessen Eigensteifigkeit dieses Erfordernis.

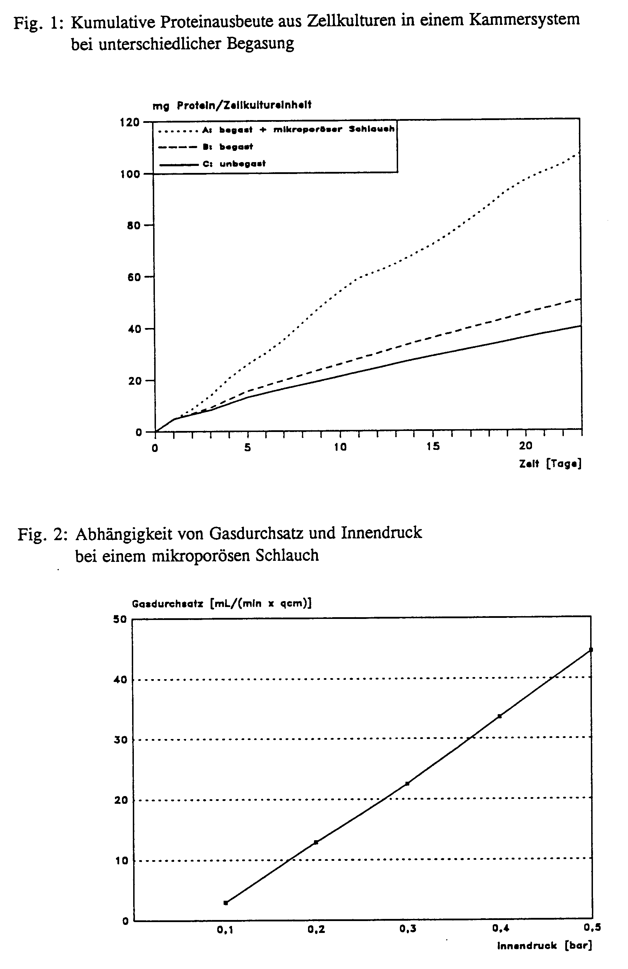

Es hat sich gezeigt, daß im Vergleich mit einem herkömmlichen Kammersystem die Zellproduktausbeute durch ein Kammersystem mit der erfindungsgemäßen Vorrichtung wesentlich gesteigert werden kann, wie im folgenden Beispiel Nr. 1 und in Figur 1 erläutert wird.

Die Erfindung wird anschließend anhand von Beispielen näher erläutert, ohne jedoch darauf beschränkt zu sein.

### Beispiel 1

Dieses Beispiel zeigt anhand eines Vergleiches die Ausbeute an erwünschtem Protein bei Verwendung eines herkömmlichen Kammersystems, eines Kammersystems mit einfacher Begasung und eines Kammersystems mit der erfindungsgemäßen Vorrichtung. In einem kommerziell erhältlichen Wannenstapel, der über 40 miteinander kommunizierende Kammern verfügt, wurde in einen der beiden Befüllungskanäle ein einseitig verschlossener, mikroporöser Schlauch eingebracht und über das offene Ende an eine Gasmischvorrichtung angeschlossen ("A"). Über eine geeignete Pumpe wurde eine definierte Menge Gas durch den Schlauch in das Kultivierungssystem gepumpt. Der Gasaustritt erfolgte über den zweiten Befüllungskanal des Wannenstapels. In einem zweiten Ansatz wurde ein mit dem vorhin beschriebenen identischer Wannenstapel ohne die erfindungsgemäße Vorrichtung eingesetzt. Die Begasung wurde hier lediglich durch Einpumpen des Gasgemisches in die Befüllungsöffnung bewirkt ("B"). Ein dritter, unbegaster Wannenstapel diente als Kontrolle ("C"). In allen drei Wannenstapeln wurde eine Kultur von gentechnisch veränderten Zellen kultiviert und die Menge des synthetisierten Proteins in definierten Abständen gemessen. Die Ergebnisse sind in Figur 1 dargestellt.
Wie die Ergebnisse zeigen, produziert der Wannenstapel ("A") mit der erfindungsgemäßen Vorrichtung im gleichen Zeitraum eine um ca. 2-fach höhere Menge an Protein als ein begaster Wannenstapel ohne die erfindungsgemäße Vorrichtung ("B"). Gegenüber einem unbegasten Wannenstapel ("C") betrug die Ausbeute etwa das 3-fache.

### Beispiel 2

Für dieses Beispiel wurde ein handelsüblicher mikroporöser Schlauch mit einem Innendurchmesser von 2 mm, einer Wandstärke von 0,4 mm und einer Porenweite von etwa 1,2 µm an einem Ende verschlossen und am anderen Ende an eine Luftdruckquelle angeschlossen. Die über die Poren des Schlauches austretende Luftmenge wurde mit Gasdurchflußmessern quantifiziert und in Fig. 2 graphisch dargestellt.

### Beispiel 3

In Fig. 3 ist der schematische Aufbau eines geschlossenen Begasungssystems dargestellt, bei dem das über eine Meß- und Regeleinheit überwachte und eingestellte Gasgemisch durch das Kammersystem zirkuliert und nur der verbrauchte Sauerstoff ersetzt, sowie das überschüssige Kohlendioxid ausgetragen wird.
Dafür ist das Kammersystem mit zwei Meßsonden für den im Medium gelösten Sauerstoff (2) und den Säuregrad (1) versehen. Die Meßdaten werden an eine handelsübliche Steuerungseinheit übermittelt, die aufgrund vorprogrammierter Grenzwerte über Magnetventile (3,4,5) eine Zudosierung von Sauerstoff (O₂), Stickstoff (N₂) und Kohlendioxid (CO₂) vornimmt. Im Falle eines sehr hohen Sauerstoffverbrauchs oder bei besonders starker Ansäuerung der Kulturen kann die Regeleinheit durch Erhöhung des Gasflusses, der über einen Meßfühler (6) registriert wird, die Leistung der Membranpumpe (9) erhöhen und so eine Verbesserung des Gasaustausches erzielen. Der erfindungsgemäße mikroporöse Schlauch ist in einen der beiden Befüllungskanäle eingeschoben und direkt mit dem Zirkulationssystem verbunden. Der durch die Zudosierung entstehende Systemüberdruck wird durch ein entsprechend eingestelltes Überdruckventil (10) auf 0,2 bar begrenzt. Da bei der raschen Entleerung und Befüllung des Kammersystems mit Zellkulturmedium ein wesentlich schnellerer Druckausgleich erforderlich ist, als er über das mikroporöse Schlauchsystem erfolgen kann, wurde zusätzlich ein Bypass (7) vorgesehen, der über ein Dreiwegeventil (8) bei Bedarf auch einen schnellen Druckausgleich erlaubt. Mit dem beschriebenen Aufbau konnte sowohl die Sauerstoffkonzentration wie auch der Säuregrad des Mediums bei der Kultivierung verschiedener Zellinien in engen Grenzen konstant gehalten werden, wie im Beispiel 4 näher ausgeführt.

### Beispiel 4

Bei dem in Beispiel 3 beschriebenen, geschlossenen System wurde über die Meßsonden 1 und 2 die Konzentration von gelöstem Sauerstoff und der Säuregrad (pH-Wert) im Nährmedium kontinuierlich gemessen und aufgezeichnet. In Fig. 4 wird dem Verlauf der Sauerstoffsättigung in dem geschlossenen und begasten System jener in einem vergleichbaren offenen und unbegasten System gegenübergestellt. Fig. 5 zeigt den entsprechenden Verlauf der pH-Werte. Es kann deutlich abgelesen werden, daß bei dem erfindungsgemäßen Verfahren eine hohe Gleichmäßigkeit der Sauerstoffkonzentration, sowie ein Anheben des pH-Wertes in den gewünschten Bereich erzielt wird, während bei dem unbegasten, offenen System ein ungünstiger Verlauf der Sauerstoffkonzentration und des pH-Wertes zu erkennen ist.

### Beispiel 5

In einen Wannenstapel mit 40 Kultivierungskammern und dem erfindungsgemäßen Begasungssystem wurde ein mit dem pH-Indikator Phenolrot versetztes, säureneutrales Zellkulturmedium eingebracht und mit reinem Kohlendioxid begast. Parallel dazu wurde ein zweiter Wannenstapel ohne die erfindungsgemäße Vorrichtung mit der gleichen Menge von Kohlendioxidgas durchströmt. Durch den partiellen Übertritt des Kohlendioxides in das Medium ergibt sich eine zunehmende Ansäuerung damit eine Verfärbung des Indikators von rot nach gelb. Während in dem Wannenstapel ohne erfindungsgemäße Vorrichtung nach 20-minütiger Durchströmung eine Gelbfärbung nur in den oberen beiden Kammern beobachtet werden konnte, zeigte der Wannenstapel mit der erfindungsgemäßen Vorrichtung eine gleichmäßige Verfärbung aller Kammern.

## Patentansprüche

1. Verfahren zur Kultivierung von Zellen höherer Organismen in kommunizierenden Kammersystemen, bei dem man die zu kultivierenden Zellen in das Kammersystem einbringt und die Züchtung durchführt, dadurch gekennzeichnet, daß man die erforderlichen Kulturbedingungen durch gleichmäßige Oberflächenbegasung mittels einer in das Kammersystem eingebrachten mikroporösen Gasversorgungsleitung erzielt.

2. Verfahren nach Anspruch 1, bei dem man die Begasung in einem geschlossenen System bewirkt, in dem das Gasgemisch zirkuliert wird.

3. Vorrichtung zur Kultivierung von Zellen höherer Organismen in kommunizierenden Kammersystemen, dadurch gekennzeichnet, daß die Vorrichtung eine mikroporöse Gasversorgungsleitung aufweist.

4. Vorrichtung nach Anspruch 3, dadurch gekennzeichnet, daß die mikroporöse Gasversorgungsleitung eine Hohlmembran in Form eines Schlauches oder Stabes ist.

5. Vorrichtung nach Anspruch 4, dadurch gekennzeichnet, daß diese mikroporöse Gasversorgungsleitung eine mikroporöse Polypropylen- oder Teflonmembran ist.

6. Vorrichtung nach einem der Ansprüche 1 und 5, dadurch gekennzeichnet, daß die mikroporöse Gasversorungsleitung durch eine Umgehungsleitung (Bypass) für den Druckausgleich zur schnellen Entleerung oder Befüllung des Kammersystems versehen ist.
